# EUROPEAN PATENT APPLICATION

(11) **EP 4 230 059 A1**
(43) Date of publication of application: **23.08.2023**
(21) Application number: 23156256.2
(22) Date of filing: 13.02.2023
(51) Int. Cl.: A23L 33/105, A23L 33/115, A23L 33/145, A23L 33/15, A23L 33/175, A61K 31/202, A61K 31/355, A61K 36/06, A61P 3/06, A61P 3/10, C12N 1/14, A61K 31/575, A61K 36/899, A23L 33/12, A23L 7/104, A61K 36/062

(54) **COMPOSITION FOR THE CONTROL OF HYPERLIPIDAEMIA**

(30) Priority: 21.02.2022 IT 202200003191
(71) Applicant: Inpha Research S.r.l., 20122 Milano MI (IT)
(72) Inventor: Castelli, Simone, Milano MI (IT); Samaritani, Giuseppe, Milano MI (IT); Fratter, Andrea, Milano MI (IT)
(74) Representative: Perani & Partners S.p.A.

(57) **Abstract**

Solid composition comprising A) a solid fraction containing: a fermented red rice extract having a total monacolin titre comprised between 1% and 5% by weight of the total weight of the fermented red rice extract, coenzyme Q10, basic L-arginine, a bulking agent selected from the group consisting of at least one polyol, maltodextrins, polydextrose, fructooligosaccharides, polysaccharides or mixtures of the foregoing; B) a liquid fraction which contains: at least one mono- or polyunsaturated fatty acid having free carboxylic function, a plant extract containing tocotrienols and tocopherols, having a tocotrienol and tocopherol titre comprised between 50% and 75% by weight of the total weight of the plant extract.

## Description

### FIELD OF THE INVENTION

The field of the present invention relates to a solid composition and relative use and preparation method, comprising active ingredients useful in the treatment of hyperlipidaemia.

### BACKGROUND ART

Red Yeast Rice (RYR) extract is a plant extract obtained by fermenting red rice in the presence of Monascus purpureus; the RYR expresses Monacolin in its phytocomplex, including Monacolin K. In particular, Monacolin K (or MoK) carries out a cholesterol-lowering action, being structurally identical to the synthesis statin Lovastatin. Lovastatin is a lactone which is activated into its active form β-hydroxy-carboxylic (pro-drug) following hydrolysis by specific hydrolases (serum and tissue lactases). Such substances, which have been the mainstay of cholesterol-lowering therapy for decades, inhibit HMG-CoA reductase, a key enzyme in the synthesis of Mevalonate, a precursor to cholesterol and to coenzyme Q₁₀ (CoQ₁₀).

Due to this peculiar pharmacodynamic and structural analogy with Lovastatin, the Monacolin K comprised in fermented red rice extract is, to date, one of the most used plant substances in food supplements for the purpose of normalizing plasma lipids.

Another active substance known to be effective in dyslipidemias or related diseases is represented by natural mixtures of tocotrienols and tocopherols. In particular, tocotrienols are molecules structurally linked to tocopherol and extractable in nature from different plant species in the form of oils.

### Problem of the prior art

Despite the now-consolidated activity and relative effectiveness, a future regulatory restriction is expected regarding the use of the Monacolin expressed in fermented red rice extract due to alleged adverse effects of a muscular nature typical of statins. In fact, it seems that a number of patients experience muscle pain of a more or less severe degree and of variable duration during the daily use of fermented red rice extract products and at the dosage of Monacolin K of 10 mg (1). In the light of this and of the likely forthcoming dosage restrictions of fermented red rice extract imposed by the parliamentary bodies (EFSA, Ministry of Health), it seems quite necessary to supplement this dosage reduction, therefore also likely to be effective, with associations of active ingredients and carrier technologies, capable of improving the effectiveness of the fermented red rice extract at the same dosage.

Monacolin is also classified as BCS class 2 (Biopharmaceutics Classification System), i.e., with good permeability, but poor solubility. Their delivery in the form of RYR actually increases its hydro-dispersibility and reduces its crystallinity, favouring an increase in its oral bioaccessibility as described by Chen et al. (1, 2). However, RYR extract containing different substances, including tannins, proteins, polysaccharides and fibre, can hinder the full bioaccessibility of the monacolins.

The low bioavailability of coenzyme Q₁₀, quinone with isoprene chain, is mainly due to its hydrophobicity and high molecular weight. However, a better bioavailability of CoQ₁₀ could be achieved using solubilized/emulsified forms (including soft gels or liquid preparation forms), colloidal systems or oily suspensions (including soft gels), although these proved to be less effective with respect to the solubilized forms (4-10). In addition, in the context of dyslipidemias or related diseases, a reduction or depletion of CoQ₁₀ associated with the prolonged use of statins in muscle tissue and plasma is observed (3). CoQ₁₀ is known to be a fundamental mitochondrial factor of the respiratory chain and its synthesis is in fact governed by HMG-CoA reductase, inhibited, as mentioned, precisely by statins and therefore also by MoK (3).

Therefore, there is a need to develop compositions with novel carrier technologies capable of increasing the bioavailability and bioaccessibility, preferably in the enteral context, of the above active ingredients, especially when administered in combination.

### SUMMARY OF THE INVENTION

A first object of the present invention is a solid composition comprising
A) a solid fraction containing:
   - a fermented red rice extract having a total monacolin titre comprised between 1% and 5% by weight of the total weight of the fermented red rice extract,
   - coenzyme Q₁₀,
   - basic L-arginine,
   - a bulking agent selected from the group consisting of: at least one polyol, maltodextrin, polydextrose, fructooligosaccharides, polysaccharides or mixtures thereof;
B) a liquid fraction which contains:
   - at least one mono or polyunsaturated fatty acid having at least one free carboxylic function,
   - a plant extract containing tocotrienols and tocopherols, having a tocotrienol and tocopherol titre of between 50% and 75% by weight of the total weight of the plant extract.

Preferably, the solid composition is for medical use, as a food supplement or as a food for special medical purposes; preferably the composition is for use in the prevention and/or treatment of a disease or disorder selected from the group consisting of: dyslipidemia, primary or secondary hypercholesterolemia, primary or secondary hypertriglyceridemia, metabolic syndrome, cardiovascular disease and combinations thereof.

A further object of the invention is a method for preparing the solid composition comprising the following phases:
a. preparing the fermented red rice extract, coenzyme Q₁₀, basic L-arginine and at least one polyol to obtain a solid preparation,
b. mixing the mixture of tocotrienols and tocopherols and the at least one mono or polyunsaturated fatty acid to obtain a liquid preparation,
c. combining the solid preparation with the liquid preparation at room temperature, possibly adding suitable excipients and/or diluents, to obtain the solid composition.

### Advantages of the invention

The solid composition of the invention is capable of solving the problems of the state of the art related to a low or reduced bioavailability of the active ingredients, such as fermented red rice extract, coenzyme Q₁₀ and the mixture of tocotrienols and tocopherols.

In fact, the composition of the invention allows the delivery of the active ingredients in a highly assimilable form by the duodenal enteric epithelium thanks to the prompt emulsification and hydro-dispersion triggered upon contact with the gastro-enteric fluids.

In particular, the at least one mono- or polyunsaturated fatty acid forms a salt with the basic L-arginine. Consequently, this promotes the formation of an extemporaneous and *in situ* emulsion, which is capable of hydro-dispersing the mixture of tocotrienols and tocopherols, and incorporating the fermented red rice extract and CoQ₁₀ into mixed micelles, increasing their enteric bioaccessibility.

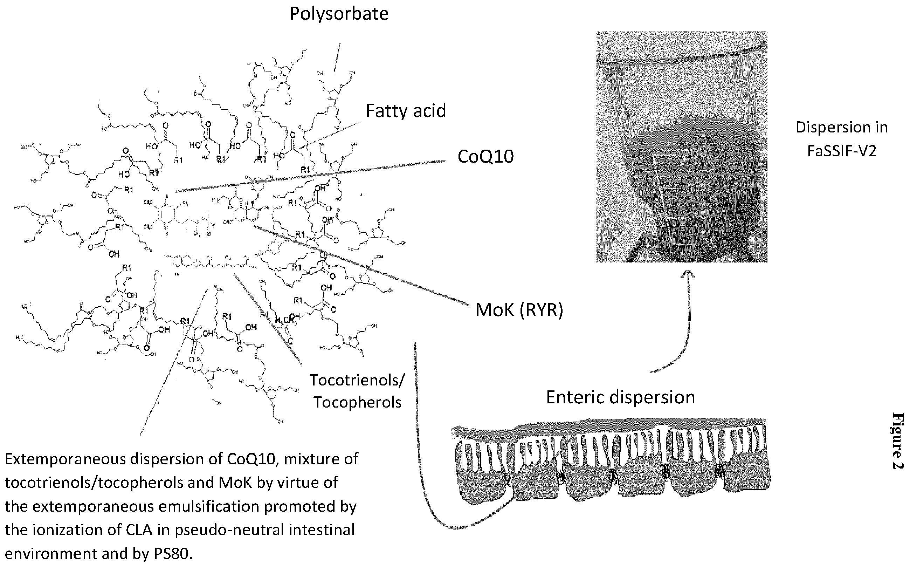

In order to obtain a prompt gastro-enteric dispersion of the aforesaid substances and at the same time to obtain an even better dissolution of the RYR, a technology was designed capable of promoting these phenomena by creating an extemporaneous emulsion (Figure 2).

### DESCRIPTION OF THE FIGURES

**Figure 1** - Comparison between a sample (1) with the composition of the invention and a sample (2) with the same composition but without the at least one fatty acid and the basic L-arginine referred of Example 4.
**Figure 2** - Representation of the enteric dispersion of fermented red rice extract, coenzyme Q₁₀ and tocotrienol mixture.

### DETAILED DESCRIPTION OF THE INVENTION

The Applicant describes the invention in more detail below.

### Solid composition

### Solid fraction/liquid fraction.

The solid composition comprises a fraction of the ingredients (i.e., a part of the ingredients) in solid form and a part of the ingredients in liquid form, preferably in oily form.

Preferably, the solid fraction of the ingredients constitutes between about 85% and 95% by weight, preferably between about 87% and 93% by weight, preferably between about 90% and 93% by weight, preferably equal to about 90%, 91%, 92%, 93% by weight of the total weight of the composition.

According to the preferred embodiment, the weight ratio liquid fraction:solid fraction varies between about 1:5 and 1:14, preferably between about 1:6 and 1:14, preferably between about 1:7 and 1:14, preferably equal to about 1:7, 1:8, 1:9, 1:10, 1:11, 1:12, 1:13, 1:14.

According to a preferred embodiment, the solid fraction comprises a solid carrier and solid active ingredients.

It should be noted that, preferably, the solid composition comprises excipients and/or diluents suitable for making a formulation for oral intake; such excipients and/or diluents preferably constitute a part of the solid fraction of the composition according to the invention.

Still preferably, the solid active ingredients and the liquid fraction of the composition are in a weight ratio of about 2:1 to 1:2; preferably about 1.6:1 to 1:1.5; preferably about 1.6:1; 1:1; 1:1.5; 1:1.2.

### Active ingredients

The solid composition comprises active ingredients which in turn comprise or consist of a fermented red rice extract, coenzyme Q₁₀, a mixture of tocotrienols and tocopherols obtained from a plant extract.

According to a preferred embodiment, the solid composition of the invention comprises, as the only active ingredients, a fermented red rice extract, coenzyme Q₁₀, and a mixture of tocotrienols and tocopherols obtained from a plant extract.

The fermented red rice extract is preferably obtained by suitable fermentation of the rice by the microorganism *Monascus purpureus*; thereby, the final extract is enriched with substances structurally and/or functionally similar to statins.

Fermented red rice extract has a total Monacolin titre comprised between 1% and 5% by weight, preferably comprised between 2% and 4% by weight, preferably 3% by weight, of the total weight of the fermented red rice extract.

The total Monacolin contained in the fermented red rice is varied. The titre in total monacolins represents the sum of each of them, but is conventionally expressed as monacolin K. Monacolin K (lactone and acid) is predominant and represents about 70-80% of the total monacolins.

Preferably, the fermented rice extract is in an amount comprised between about 4% and 11% by weight, preferably comprised between about 5% and 10% by weight, preferably comprised between about 7% and 9% by weight, preferably comprised between about 6% and 9% by weight, preferably equal to about 6%, 7%, 8%, or 9% of the total weight of the composition.

Preferably, the absolute amount of Monacolin in the solid composition is comprised between 1 mg and 10 mg, preferably comprised between 1.5 and 7 mg, preferably comprised between 1.5 mg and 5 mg, preferably comprised between 1.5 mg and 4 mg, preferably comprised between 1.90 mg and 3.5 mg.

It should be noted that fermented red rice extract is an active ingredient in solid form.

Coenzyme Q₁₀ is preferably in an amount comprised between about 0.5% and 3% by weight, preferably comprised between about 0.7% and 2.5% by weight, preferably comprised between about 0.9% and 2.0% by weight of the total weight of the composition.

It should be noted that coenzyme Q₁₀ is an active ingredient in solid form.

The plant extract containing tocotrienols and tocopherols is characterized by a tocotrienol and tocopherol titre comprised between 50% and 75% by weight, preferably comprised between 60% and 75% by weight of the total weight of the plant extract.

It should be noted that the plant extract containing tocotrienols and tocopherols consists substantially of a mixture of tocotrienols and tocopherols (mainly α-, β-, γ-, δ-tocotrienols; α-tocopherol), and is preferably in liquid form, preferably in oily form.

It should be noted that natural vitamin E consists of four isomers of tocotrienols (α, β, γ, and δ) and four isomers of tocopherols (α, β, γ, and δ). Natural vitamin E is found in the seeds of plants to protect the latter from oxidation and from UV damage. Most of these seeds contain only α-tocopherol; only some seeds contain both forms.

The mixture of tocotrienols and tocopherols comprised in the composition of the invention preferably has a tocotrienol:tocopherol ratio, more preferably tocotrienols: α-tocotrienol, of about 3:1, preferably about 2.8:1.

For the purposes of the invention, the plant extract containing tocotrienols and tocopherols is preferably selected from the group consisting of wheat germ extract, carrot extract, palm extract and rice extract, more preferably it is palm extract.

When the plant extract is palm extract, the mixture of tocotrienols and tocopherols is referred to as "*Tocotrienol-Rich Fraction*" (TRF); the TRF of a palm extract preferably contains about 25% α-tocopherol and about 75% tocotrienols.

The mixture of tocotrienols and tocopherols is preferably in an amount comprised between about 2% and 15% by weight, preferably comprised between about 4.5% and 10% by weight, preferably comprised between about 5% and 9% by weight, preferably about 5%, 6%, 7%, 8%, 9% by weight of the total weight of the composition.

### Bioavailability promoters

For the purposes of the invention, bioavailability promoters are substances or combinations of substances capable of increasing the bioavailability, preferably enteral, of the active ingredients contained in the composition of the invention. For purposes of the invention, an example of a bioavailability promoter is an emulsifying agent.

The solid composition comprises bioavailability promoters which in turn comprise or consist of at least one mono- or polyunsaturated fatty acid having at least one carboxylic function in free form.

It should be noted that, in free carboxylic form, the at least one mono- or polyunsaturated fatty acid does not constitute - as such - an efficient emulsifying agent; its emulsifying power will be elicited in contact with gastro-enteric fluids, where - thanks to the formation of a salt with L-arginine - it will make an extemporaneous emulsion *in situ.*

Preferably, the at least one fatty acid and the L-arginine are combined in a molar ratio preferably comprised between 1:1 and 1:2; preferably comprised between 1:1 and 1:1.8; preferably comprised between 1:1.3 and 1:1.7; preferably comprised between 1:1.3 and 1:1.6; preferably comprised between 1:1 .3 and 1:1.5. It should be noted that the fatty acid:L-arginine molar ratio is to be understood as the molar ratio of the L-arginine to the one or each fatty acid contained in the composition.

It should be noted that the at least one mono- or polyunsaturated fatty acid is in liquid form; preferably it is a monocarboxylic fatty acid.

The at least one mono- or polyunsaturated fatty acid preferably has a number of carbon atoms comprised between 14 and 20, preferably comprised between 14 and 18, preferably comprised between 16 and 18.

Still preferably, when the fatty acid is poly-unsaturated, the number of unsaturations is comprised between 2 and 4, preferably equal to 2 or 3.

The at least one mono- or polyunsaturated fatty acid is in an amount comprised between about 1% and 5% by weight, preferably comprised between about 1.5% and 3.6% by weight, preferably equal to about 1.5%, 1.8%, 2.0%, 2.27%, 3.0%, 3.63% by weight of the total weight of the composition.

The at least one fatty acid is selected from the group consisting of: myristoleic acid, palmitoleic acid, oleic acid, linoleic acid, conjugated linoleic acid, alpha-linolenic acid (ALA), gamma-linolenic acid (GLA), and combinations of the foregoing.

According to a preferred form, the at least one fatty acid is conjugated linoleic acid.

The term conjugated linoleic acid or CLA refers to a group of positional and geometric isomers of linoleic acid (18: 2Δ9c,12c), characterized by the presence of two conjugated double bonds. The positional isomers identified so far are those with double bonds at the positions 7/9, 8/10, 9/11, 10/12, 11/13 and 12/14. For each positional isomer, 4 pairs of geometric isomers are possible (cis, cis; trans, cis; cis, trans; trans, trans). Therefore, the term CLA includes a total of 24 positional and geometric isomers of linoleic acid.

According to a preferred form, the emulsifying agent further comprises at least one ester selected from: polyoxyethylenate sorbitan ester, preferably polysorbate 80, sucrose ester of fatty acids, or combinations of the foregoing.

Preferably, the at least one ester is in an amount comprised between 1% and 10%, preferably comprised between 1.8% and 8%, preferably comprised between 1.8% and 5.7%, preferably comprised between 1.8% and 4.5%, of the total weight of the composition.

Preferably, the sucrose ester of the fatty acids is characterized by an HLB (Hydrophilic-lipophilic balance) > 12, preferably > 12 and < 18, preferably > 12 and < 15.

When the emulsifying agent comprises at least one ester, the emulsifying/micellizing effect is amplified and, consequently, an improved increase in the bioavailability of the active ingredients comprised in the composition is obtained.

In particular, when the emulsifying agent comprises both the polyoxyethylene sorbitan ester and the sucrose ester of the fatty acids, a further enhancement of the emulsifying capacity or a real synergy between these two and the at least one mono- or polyunsaturated fatty acid is generated.

### Solid carrier

The solid composition further comprises a solid carrier, i.e., a substrate useful for adsorbing at least a part of the liquid fraction of the composition according to the invention. The solid carrier constitutes a fraction of the solid ingredients of the composition, preferably it constitutes between about 40% and 50% by weight of the solid fraction of the composition according to the invention.

Preferably, the solid carrier comprises:
- a bulking agent selected from among at least one polyol, maltodextrin, polydextrose, fructooligosaccharides, polysaccharides or mixtures of the foregoing. It should be noted that polysaccharides are less preferred in the purposes of the invention, due to the caloric intake associated therewith; such caloric intake therefore makes them less suitable for making compositions aimed at patients in need of low-calorie or sugar-free therapies.
- basic L-arginine.

Still preferably, the at least one polyol is selected from the group consisting of: sorbitol, maltitol, mannitol, isomalt, erythrulose and combinations of the foregoing.

Preferably, the amount of polyol is comprised between about 28% and 45% by weight, preferably comprised between about 30% and 45% by weight, preferably comprised between about 32% and 43% by weight, preferably equal to about 32%, 33%, 34%, 35%, 36%, 37%, 38%, 39%, 40%, 41%, 42%, 43% by weight of the total weight of the composition.

Basic L-arginine is an alkalizing agent comprised in the composition of the invention. Although L-arginine is understood to be the preferred alkalizing agent for the purposes of the present invention, the person skilled in the art will have no difficulty in identifying alternative alkalizing agents suitable for the reproduction of the solid composition in question.

Basic L-arginine has, for the purposes of the invention, a dual function: that of mono- or polyunsaturated fatty acid activator as emulsifying agent; and that of solid carrier to favour the adsorption of the liquid fraction of the solid composition.

The basic L-arginine is in an amount comprised between 1% and 4% by weight, preferably between 1.3% and 3% by weight, preferably between 1.3% and 2.7% by weight, preferably between 1.3% and about 2% by weight, of the total weight of the solid composition.

The solid composition of the invention can further contain excipients and/or diluents, preferably suitable for obtaining a solid composition, more preferably a powder (to be hydrodispersed upon assumption or for filling hard capsules), granulate or a tablet. Suitable excipients and/or diluents are chosen from: diluents (such as microcrystalline cellulose), binders, disintegrants (such as carboxymethylcellulose), lubricants/glidants, sliding promoting agents (such as amorphous silica), dyes, sweeteners, flavourings.

It should be noted that the solid composition of the invention does not contain glycerin, medium-chain triglycerides, TPGS (D-α-Tocopherol polyethylene glycol 1000 succinate), Macrogol (15) -hydroxystearate (also known as polyethyleneglycol (15) - hydroxystearate or as Kolliphor HS 15), or water, except for the residual content of water (or moisture) inherently contained in the ingredients of the solid fraction.

### Pharmaceutical form

For the purposes of the present invention, a solid composition is understood to be a processable composition for the purpose of making preparations which, in pharmaceutical technology, are to be considered solid (powders, granules, tablets or capsules).

Still preferably, the solid composition is in the form of a powder, granulate, tablet or hard capsule. In particular, with reference to tablets, the solid composition of the invention allows to make compact tablets of suitable hardness, preferably not less than 9 Kg, even more preferably comprised between 14 and 18 Kg.

It should be noted that the solid composition of the invention is advantageously dispersible in water. For this reason, it can be made into solid pharmaceutical forms that can be taken as such (which disperse in gastro-enteric fluids) or forms to be dispersed in water before administration.

Still preferably, the solid composition of the invention is in the form of a swallowable tablet or hydrodispersible powder.

It should be noted that the solid composition cannot be made into soft capsules (or soft gels).

The solid composition of the invention is preferably taken orally.

The solid composition is preferably in the form of a food supplement, food for special medical purposes (FSMP), pharmaceutical product (medicament).

Food supplement means a formulation which falls under the definition of Directive 2002/46/EC and subsequent amendments. In this legislation, food supplements are precisely defined as: *"foodstuffs intended to supplement the common diet and constituting a concentrated source of nutrients, such as vitamins and minerals, or of other substances having a nutritional or physiological effect, in particular, but not exclusively, amino acids, essential fatty acids, fibres and extracts of plant origin, both mono- and multi-compound, in predominant forms"*.

Food for Special Medical Purposes (or FSMP) means food products expressly processed or formulated for the dietary management of patients with specific nutritional needs, including infants, to be used "under medical supervision". FSMPs are intended for the complete or partial feeding of patients who have a limited or altered ability to take, digest, absorb, metabolize or eliminate common foods or certain nutrients contained therein, or who have specific nutritional needs determined by clinical conditions. FSMPs differ from other foods and food supplements themselves in certain characteristics, such as: they require medical supervision; they can replace - totally or in part - normal nutrition; they are indicated for specific pathological conditions. As for the composition, FSMPs are regulated by Regulation 609/13.

### Therapeutic use of the solid composition

Preferably, the solid composition of the invention is intended for medical use (as a medicament/medicinal product); or for food supplementation. It should be noted that the solid composition in food supplement/FSMP form can also be intended for use as an adjuvant to traditional lipid-lowering drug therapies.

Still preferably, the solid composition is for use in the prevention and/or treatment of hyperlipidaemias. Preferably, the hyperlipidaemias, in the purposes of the present invention, are a disease or a disorder selected from the group consisting of: dyslipidemia, primary or secondary hypercholesterolemia, primary or secondary hypertriglyceridemia, metabolic syndrome, cardiovascular pathology, and combinations of the foregoing.

Prevention is intended an activity aimed at reducing mortality, morbidity or effects due to certain risk factors or a certain disease (prophylaxis), promoting individual and collective health and well-being.

For the purposes of the invention, cardiovascular disease means an acute or chronic disease associated with or induced by altered blood concentrations of triglycerides and/or cholesterol; by way of non-limiting example, it includes coronary artery disease, atherosclerosis, hypertension, myocardial infarction.

### Method for preparing the solid composition

As already explained above, the method for preparing the solid composition comprises the following phases:
a. preparing the fermented red rice extract, coenzyme Q₁₀, basic L-arginine and at least one polyol to obtain a solid preparation,
b. mixing the mixture of tocotrienols and tocopherols and the at least one mono or polyunsaturated fatty acid to obtain a liquid preparation,
c. combining the solid preparation with the liquid preparation at room temperature, possibly adding suitable excipients and/or diluents, to obtain the solid composition.

It should be noted that, preferably, the liquid preparation is in oily form.

The solid preparation prepared in step (a) of the method corresponds to the solid fraction of the composition according to the invention.

According to a preferred embodiment, the solid preparation comprises
- a first solid preparation, obtained by mixing the fermented red rice extract and coenzyme Q₁₀. Preferably the first solid preparation comprises or consists of the solid active ingredients;
- a second solid preparation, obtained by mixing the L-Arginine and the polyol, separately from the first solid preparation. Preferably, the second solid preparation comprises or consists of the solid carrier.

Still preferably, the phase (c) comprises the sub-phases of:
- wetting the first solid preparation with the liquid preparation to obtain a wet solid preparation (or paste),
- mixing the wet solid preparation (or paste) with the second solid preparation, possibly adding suitable excipients and/or diluents, to obtain the solid composition.

For the purposes of the invention, room temperature means a temperature comprised between 20°C and 28°C, preferably comprised between 20°C and 25°C, preferably equal to 20°C.

Preferably, each phase or sub-phase of the method of the invention occurs at room temperature. In this sense, the method of the invention is advantageous because it avoids the use of high temperatures, which could modify or degrade certain active ingredients; moreover, the process of preparing the composition of the invention is simplified.

According to a preferred form, the method for preparing the solid composition of the invention comprises the following phases:
a. combining and mixing the fermented red rice extract and coenzyme Q₁₀ to obtain the first solid preparation, sieving if necessary;
b. combining and mixing the mixture of tocotrienols and tocopherols and the at least one mono or polyunsaturated fatty acid to obtain the liquid preparation,
c. mixing the basic L-arginine and the at least one polyol to obtain the second solid preparation, possibly sieving;
d. wetting the first solid preparation with the liquid preparation to obtain a wet solid preparation (or paste);
e. combining the second solid preparation with the wet solid preparation at room temperature and mixing, preferably under manual or mechanical stirring, optionally adding suitable excipients and/or diluents, optionally sieving, to obtain the solid (non-wet) composition of the invention.

### EXAMPLES

Below the Applicant reports examples merely for illustrative and non-limiting purposes.

### Example 1 - Solid composition

| **Phase** | **Raw material** | **Quantity (g)** |
|---|---|---|
| A | Fermented red rice extract 3% Monacolin | 0.100 |
| A | Coenzyme Q₁₀ | 0.010 |
| B | DAVOS LIFE E3 DVL 50 - Mixture of tocotrienols and tocopherols (tit. 50%) | 0.050 |
| B | CLARINOL FFA 80 (TONALIN FFA 80) (PM=280.4) - CLA | 0.020 |
| C | Basic L-arginine (MW=174.2) | 0.015 |
| C | Sorbitol | as needed **1.1 g** |
| D | Amorphous silica | 0.050 |
| E | Microcrystalline cellulose | 0.300 |
| D | Calcium phosphate | 0.050 |
| E | Carboxymethylcellulose | 0.030 |

### Method for preparing the solid composition of example 1:

Phase A: mix together the fermented red rice extract and coenzyme Q₁₀ in plastic beaker until a homogeneous powder is obtained (if necessary sieve).
Phase B: combine DAVOS LIFE with CLARINOL FFA 80 and stir until the liquid phase is homogeneous and clear (strong red colour).
Phase C: combine the L-Arginine and sorbitol together until a homogeneous white powder is obtained (if necessary sieve).

Proceed to wet Phase A with Phase B until a wet powder (paste) is obtained.

Combine Phase C on Phase A+B at room temperature under manual or mechanical stirring until a wet and homogeneous powder (granule) is obtained. Continue to sieving.

Combine Phase A+B+C with the ingredients of Phase D and E and mix until a fine, uniform, non-moist powder is obtained. Sieve.

### Example 2 - Solid composition

| **Phase** | **Raw material** | **Quantity (g)** |
|---|---|---|
| A | Fermented red rice extract 3% Monacolin | 0.0660 |
| A | Coenzyme Q₁₀ | 0.0200 |
| B | DAVOS LIFE E3 DVL 50 - Mixture of tocotrienols and tocopherols (tit. 50%) | 0.0750 |
| B | CLARINOL FFA 80 (TONALIN FFA 80) - CLA | 0.0300 |
| B | VEREMUL T 80 | 0.0200 |
| C | Basic L-arginine | 0.0223 |
| C | Sorbitol | as needed **1.1 g** |
| D | Amorphous silica | 0.065 |
| E | Microcrystalline cellulose | 0.300 |
| E | Calcium phosphate | 0.050 |
| E | Carboxymethylcellulose | 0.030 |

### Method for preparing the solid composition of example 2:

Phase A: mix together the Fermented red rice and Coenzyme Q10 in plastic beaker until a homogeneous powder is obtained (if necessary sieve).
Phase B: combine DAVOS LIFE with the CLARINOL and the VEREMUL T 80 and stir until the liquid phase is homogeneous and clear (strong red colour).
Phase C: combine the L-Arginine and sorbitol together until a homogeneous white powder is obtained (if necessary sieve).

Proceed to wet Phase A with Phase B until a wet powder (paste) is obtained.

Combine Phase C on Phase A+B at room temperature under manual or mechanical stirring until a wet and homogeneous powder (granule) is obtained. Continue to sieving.

Combine Phase A+B+C with the ingredients of Phase D and E and mix until a fine, uniform, non-moist powder is obtained. Sieve.

### Example 3 - Solid composition

| **Phase** | **Raw material** | **Quantity (g)** |
|---|---|---|
| A | Fermented red rice extract 3% Monacolin | 0.075 |
| A | Coenzyme Q₁₀ | 0.020 |
| B | DAVOS LIFE E3 DVL 50 - Mixture of tocotrienols and tocopherols 50% | 0.100 |
| B | CLARINOL FFA 80 (TONALIN FFA 80) - CLA | 0.040 |
| B | Sucrose ester (HLB > 12) | 0.050 |
| C | Basic L-arginine | 0.030 |
| C | Sorbitol | as needed **1.1 g** |
| D | Amorphous silica | 0.050 |
| E | Microcrystalline cellulose | 0.300 |
| E | Calcium phosphate | 0.050 |
| E | Carboxymethylcellulose | 0.030 |

### Method for preparing the solid composition of example 3:

Phase A: mix together the Fermented red rice and Coenzyme Q10 in plastic beaker until a homogeneous powder is obtained (if necessary sieve).
Phase B: combine DAVOS LIFE with CLARINOL and stir until the liquid phase is homogeneous and clear (strong red colour).
Phase C: combine the L-Arginine and sorbitol together with the SUCROSE ESTER until a homogeneous white powder is obtained (if necessary sieve).

Proceed to wet Phase A with Phase B until a wet powder (paste) is obtained.

Combine Phase C on Phase A+B at room temperature under manual or mechanical stirring until a wet and homogeneous powder (granule) is obtained. Continue to sieving.

Combine Phase A+B+C with the ingredients of Phase D and E and mix until a fine, uniform, non-moist powder is obtained. Sieve.

### Example 4 - FaSSIF Experiment

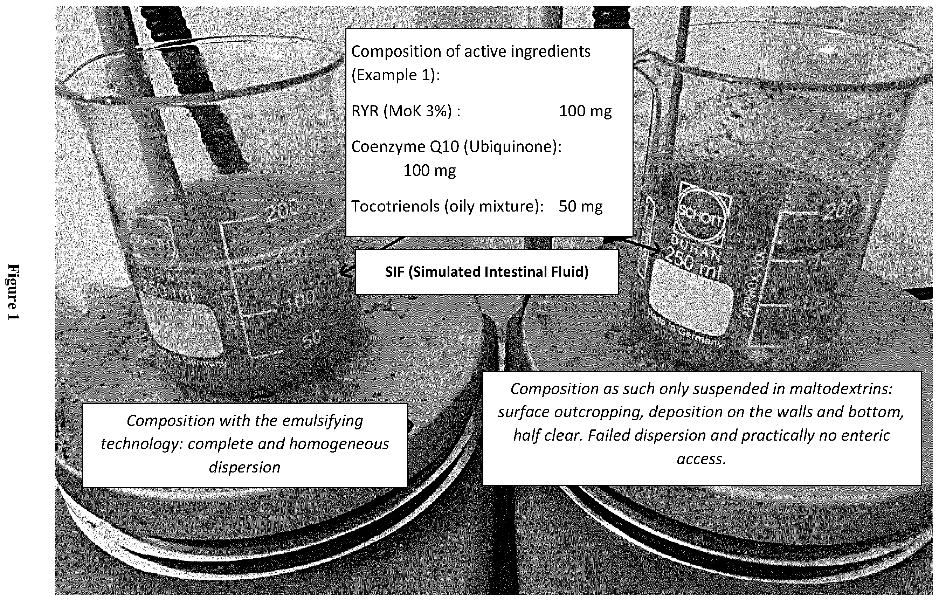

With reference to Figure 1, the Applicant carried out an experiment, dissolving two samples in Fasted State Simulated Intestinal Fluid (FaSSIF-V2) at 37°C: sample (1) - consisting of the composition of example 1, and sample (2) - consisting of the same mixture of active ingredients of sample (1) (fermented red rice, Coenzyme Q₁₀ and mixture of tocotrienols and tocopherols) suspended in maltodextrins (therefore free of fatty acid, polyol and L-arginine).

It should be noted that sample (1) is characterized by complete and homogeneous dispersion; this is indicative of optimal enteric access.

Conversely, sample (2) is characterized by incomplete and uneven dispersion, with surface emergence, deposition on the walls and bottom of the flask, half clear; this means practically no enteric access.

### REFERENCES

1. EFSA Panel on Food Additives and Nutrient Sources added to Food (ANS). Scientific opinion on the safety of monacolins in red yeast rice. EFSA J. 2018 Aug 3;16(8):e05368. doi: 10.2903/j.efsa.2018.5368;
2. Chen C-H, Yang J-C, Uang Y-S and Lin C-J, 2013. Improved dissolution rate and oral bioavailability of lovastatin in red yeast rice products. The International Journal of Pharmaceutics, 444, 18-24;
3. Hua Qu et al. The effect of statin treatment on circulating coenzyme Q10 concentrations: an updated meta-analysis of randomized controlled trials. Eur J Med Res. 2018; 23: 57. doi: 10.1186/s40001-018-0353-6;
4. Hemmi N Bhagavan, Raj K Chopra. Plasma coenzyme Q10 response to oral ingestion of coenzyme Q10 formulations. Mitochondrion. 2007 Jun;7 Suppl:S78-88. doi: 10.1016/j.mito.2007.03.003;
5. Hemmi N Bhagavan, Raj K Chopra. Coenzyme Q10: absorption, tissue uptake, metabolism and pharmacokinetics. Free Radic Res. 2006 May;40(5):445-53. doi: 10.1080/10715760600617843;
6. Guillermo López-Lluch, Jesús Del Pozo-Cruz, Ana Sánchez-Cuesta, Ana Belén Cortés-Rodríguez, Plácido Navas. Bioavailability of coenzyme Q10 supplements depends on carrier lipids and solubilization. Nutrition. 2019 Jan;57:133-140. doi: 10.1016/j.nut.2018.05.020;
7. Christiane Schulz, Ute C Obermüller-Jevic, Oliver Hasselwander, Jürgen Bernhardt, Hans K Biesalski. Comparison of the relative bioavailability of different coenzyme Q10 formulations with a novel solubilizate (Solu Q10). Int J Food Sci Nutr. Nov-Dec 2006;57(7-8):546-55.doi: 10.1080/09637480601058320;
8. Zheng-Xian Liu, Carl Artmann. Relative bioavailability comparison of different coenzyme Q10 formulations with a novel delivery system. Altern Ther Health Med. Mar-Apr 2009;15(2):42-6.
9. Li Liu, Kai Mao, Wenting Wang, Hongchun Pan, Fen Wang, Min Yang, and Hong Liu. Kolliphor® HS 15 Micelles for the Delivery of Coenzyme Q10: Preparation, Characterization, and Stability. AAPS PharmSciTech, Vol. 17, no. 3, June 2016 (# 2015).
10. Carmen J. Pastor-Maldonado, Juan M. Suárez-Rivero, Suleva Povea-Cabello, Mónica Álvarez-Córdoba, Irene Villal6n-Garcia, Manuel Munuera-Cabeza, Alejandra Suárez-Carrillo, Marta Talaverón-Rey and José A. Sánchez-Alcázar. Coenzyme Q10: Novel Formulations and Medical Trends. Int. J. Mol. Sci. 2020, 21, 8432.

## Claims

1. Solid composition comprising
A) a solid fraction containing:
- a fermented red rice extract having a total monacolin titre comprised between 1% and 5% by weight of the total weight of the fermented red rice extract,
- coenzyme Q₁₀,
- basic L-arginine,
- a bulking agent selected from the group consisting of at least one polyol, maltodextrin, polydextrose, fructooligosaccharides, polysaccharides or mixtures thereof;
B) a liquid fraction containing:
- an emulsifying agent comprising or consisting of at least one mono or polyunsaturated fatty acid having a free carboxylic function,
- a plant extract containing tocotrienols and tocopherols, having a tocotrienol and tocopherol titre comprised between 50% and 75% by weight of the total weight of the plant extract.

2. Solid composition according to claim 1, wherein the weight ratio of basic L-arginine:fatty acid is comprised between 1:1 and 1:2.

3. Solid composition according to claim 1 or 2, wherein the emulsifying agent further comprises at least one ester selected from: polyoxyethylenate sorbitan ester, preferably polysorbate 80; sucrose ester of fatty acids; or combinations of the foregoing.

4. Solid composition according to any one of claims from 1 to 3, wherein the fermented rice extract is in an amount comprised between 4% and 11% by weight of the total weight of the composition.

5. Solid composition according to any one of claims from 1 to 4, wherein the coenzyme Q₁₀ is in an amount comprised between 0.5% and 3% by weight of the total weight of the composition.

6. Solid composition according to any one of claims from 1 to 5, wherein the plant extract containing tocotrienols and tocopherols is in an amount comprised between 2% and 15% by weight of the total weight of the composition.

7. Solid composition of any one of claims from 1 to 6, wherein the liquid fraction and the solid fraction are in a weight ratio comprised between 1:5 and 1:14.

8. Solid composition according to any one of claims from 1 to 7, in form of a powder, granulate, tablet or hard capsule.

9. Solid composition according to any one of claims from 1 to 8, in an oral deglutible or hydrodispersible pharmaceutical form before administration.

10. Solid composition according to any one of claims from 1 to 9, for use as a medicine, as a food supplement or as food for special medical purposes.

11. Solid composition according to any one of claims from 1 to 10, for use in the prevention and/or treatment of hyperlipidemia.

12. Method for preparing the oral composition according to any one of claims from 1 to 9, comprising the following phases:
a. preparing the fermented red rice extract, coenzyme Q₁₀, basic L-arginine and at least one polyol to make a solid preparation,
b. mixing the extract containing tocotrienols and tocopherols and the at least one mono or polyunsaturated fatty acid to obtain a liquid preparation,
c. combining the solid preparation and the liquid preparation at room temperature, possibly adding suitable excipients and/or diluents, to obtain the solid composition.

13. The method according to claim 12, wherein each of the phases constituting the method is conducted at room temperature.
